# EUROPÄISCHE PATENTSCHRIFT

(11) **EP 1 234 050 B1**
(45) Veröffentlichungstag und Bekanntmachung des Hinweises auf die Patenterteilung: **25.04.2007**
(21) Anmeldenummer: 00985017.3
(22) Anmeldetag: 13.11.2000
(51) Int. Cl.: C12Q 1/28

(54) **NACHWEIS VON PEROXIDASEN**
DETECTION OF PEROXIDASES
DETECTION DE PEROXYDASES

(30) Priorität: 29.11.1999 DE 19957367
(43) Veröffentlichungstag der Anmeldung: 28.08.2002
(73) Patentinhaber: Merck Patent GmbH, 64271 Darmstadt (DE)
(72) Erfinder: TANZER, Dieter, 64372 Ober-Ramstadt (DE); KRENN, Karl-Dieter, 64319 Pfungstadt (DE)
(86) Internationale Anmeldenummer: PCT/EP2000/011200
(87) Internationale Veröffentlichungsnummer: WO 2001/040501

(56) Entgegenhaltungen:
- EP-A- 0 832 985
- WO-A-80/02800
- US-A- 4 755 472

## Beschreibung

Die Erfindung betrifft ein Verfahren und ein Mittel zum schnellen Nachweis von Peroxidasen in Milch und Milchprodukten.

Die thermische Behandlung nimmt entscheidenden Einfluß auf die Qualität und Hygiene des Lebensmittels Milch. Hauptaufgabe der Wärmebehandlung ist das Abtöten von pathogenen Keimen bzw. Verderbniserregern. Die Behandlung hat jedoch auch Veränderungen weiterer Milchbestandteile wie z.B. die Inaktivierung von milcheigenen Enzymen zur Folge. Die Erhitzungsbedingungen müssen daher so eingestellt werden, daß erwünschte Eigenschaften im Endprodukt erreicht werden, ohne gleichzeitig unerwünschte Nebeneffekte zu erzeugen.

Die Temperatur-Zeit-Bereiche zur Herstellung der verschiedenen Milchsorten sind in der bundesdeutschen Milchverordnung festgelegt (Verordnung über Hygiene- und Qualitätsanforderungen an Milch und Erzeugnisse auf Milchbasis (Milchverordnung) vom 24. April 1995 (in H. Loos und T. Nebe: Das Recht der Milchwirtschaft in der Bundesrepublik Deutschland, Babd V, S. 27-28, Behr's Verlag, Hamburg (130. Ergänzungslieferung vom 31.12.97)). Pasteurisierung, Ultrahocherhitzung (UHT) und Sterilisierung sind anerkannte Wärmebehandlungsverfahren. Dauererhitzung, Kurzzeiterhitzung und Hocherhitzung sind weitere Unterscheidungskriterien für pasteurisierte Milch.

Zur Kontrolle und Charakterisierung wärmebehandelter Milch macht man sich unter anderem die temperaturabhängige Inaktivierung milcheigener Enzyme zu Nutze (M. W. Griffiths, J. Food Protection, 49 (1986), S. 696 - 705; M. Villamiel et al., Z. Lebensm. Unters. Forsch. 208 (1999), S. 169-171). Während der Nachweis der alkalischen Phosphatase zur Kontrolle einer genügenden Dauererhitzung oder Kurzzeiterhitzung eingesetzt wird, ermöglicht die Messung der milcheigenen Peroxidase (Lactoperoxidase) eine Beurteilung hinsichtlich der ordnungsgemäßen Hocherhitzung. Demnach muß in kurzzeiterhitzter Milch die Aktivität des originären Milchenzyms Alkalische Phosphatase negativ, die Aktivität des Milchenzyms Lactoperoxidase positiv sein, in hocherhitzter Milch müssen beide Enzyme inaktiviert sein.

Durch technische Defekte an Anlagen kann es außerdem bei der Wärmebehandlung zu einer Vermischung von wärmebehandelter Milch bzw. wärmebehandelten Milchprodukten mit ungenügend erhitzter Milch bzw. ungenügend erhitzten Milchprodukten kommen, was mit geeigneten Testverfahren überprüft werden muß.

Die steigende Bedeutung der Qualitätskontrolle hinsichtlich der thermischen Behandlung von Milch wird mit dem Inkrafttreten der europäischen Richtlinie 92/46 deutlich. Voraussetzung für eine funktionierende Qualitätskontrolle und die Definition von Grenzwerten ist die Existenz geeigneter Analysenmethoden, die eine rasche und zuverlässige Überprüfung qualitätsrelevanter Kriterien bzw. die Übereinstimmung mit der Warendeklaration (pasteurisierte Milch / UHT-Milch) ermöglichen.

Die Bestimmung der Aktivität der milcheigenen Enzyme erfolgt in der Regel mittels Farbreaktionen. Die Enzyme katalysieren begleitet von einer Farbänderung die Umsetzung (Redoxreaktionen, Hydrolysen) geeigneter Substrate. Mittels visueller oder spektrometrischer Verfahren kann eine qualitative, halbquantitative oder quantitative Auswertung der Farbänderung vorgenommen werden.

Zur Bestimmung der Aktivität der Lactoperoxidase sind im Stand der Technik verschiedene Verfahren bekannt. Als Substrate werden beispielsweise Guajakol, 1,4-Phenylendiam, N,N-dimethyl-p-phenylendiamindihydrochlorid, o-Tolidin, o-Dianisidin, 2,2'-azino-di-(3-ethylbenzthiazolin-6-sulfonsäure), 2,7 Diaminofluoren, Benzidin und 3,3',5,5'-Tetramethylbenzidin eingesetzt. Amtliche Zulassung als Nachweisreagenz (Substrat) für die ordnungsgemäße Pasteurisierung der Milch durch Hocherhitzung besitzen nur wenige Reagenzien, wie Reagenzien auf Basis von Guajakol und Phenylendiamin. Beispiele sind Guajakreagenz Neu^{®}, Hocherhitzungsreagenz N3^{®} und Traventol^{®}.

Nachteil dieser Methoden ist, daß sie naßchemisch durchgeführt werden müssen, das heißt, daß die Nachweisreaktion in Lösung ausgeführt werden muß. Die Probe wird dabei mit mehreren, teilweise giftigen Reagenzlösungen versetzt, was entsprechende Handhabungs- und Entsorgungsprobleme nach sich zieht.

In jüngerer Zeit hat die Analyse mit festen, saugfähigen Trägern, den sogenannten Teststäbchen, zunehmend an Bedeutung gewonnen. Zu den wesentlichen Vorteilen dieser trockenchemischen Verfahren gehören insbesondere die einfache Handhabung sowie die aufgrund der geringen Reagenzmengen unproblematische Entsorgung. Die meisten oder bevorzugt sogar alle der für die Nachweisreaktion notwendigen Reagenzien sind dabei in entsprechenden Schichten eines festen, saugfähigen oder quellbaren Trägers eingebettet, auf den die Probe aufgebracht wird. Nach Kontakt der Reaktionszone mit der Probe läuft die Nachweisreaktion ab. Die gebildete Farbe ist ein Maß für die Menge bzw. Aktivität des zu bestimmenden Analyten und kann visuell oder mit einfachen Reflektometern ausgewertet werden.

Aus der Literatur ist eine Vielzahl an unterschiedlichen, auch trockenchemischen Verfahren, zur Aktivitätsbestimmung von Peroxidasen und anderen peroxidatisch wirksamen Substanzen bekannt. Die Bestimmung ist für die biochemische Analytik und medizinische Diagnostik, insbesondere in Verbindung mit Immunoassays (Peroxidase-Markierung) von großer Bedeutung (K.M. Pruitt et al., Anal. Biochem. 191 (1990), 278-286).

Beispielsweise wird in WO 80/02800 der Nachweis von Östrogeninduzierter Peroxidase offenbart. Der Nachweis erfolgt mithilfe eines chromogenen Peroxidase-Substrates. Der Zusatz von Halogeniden wird als vorteilhaft für die Farbentwicklung beschrieben.

Zum Nachweis von Lactoperoxidase ist dagegen kein Verfahren bekannt, das den vorgenannten Ansprüchen bezüglich Schnelligkeit und Empfindlichkeit genügt. Die existierenden Verfahren zum Nachweis von Lactoperoxidase in Milch erweisen sich als wenig bzw. ungeeignet für eine rasche und zuverlässige Qualitätskontrolle, da sie meist an mehreren der folgenden Nachteile leiden:
- Erforderliche Reagenzien müssen in genau definierten Konzentrationen nach einer Handhabungsvorschrift zusammengegeben werden, woraus ein hoher zeitlicher Aufwand resultiert.
- Umgang mit teilweise nicht lagerstabilen und deshalb regelmäßig frisch herzustellenden Reagenzlösungen (geringe Praktikabilität, Entsorgungsprobleme).
- Insbesondere bei photometrischer Auswertung ist ein hoher apparativer Aufwand ,verbunden mit hohen Analysekosten, erforderlich.
- Geschultes Personal und geeignete Laborausstattung erforderlich.
- Störungen der Messung durch die Matrix Milch (Eigenfärbung und Trübung) erfordert zusätzliche Probenvorbereitung mit der Gefahr von falsch negativen Befunden oder Peroxidase-Minderbefunden (keine vollständige Wiederfindung).
- Aufwendige Kalibration bei visuellen Methoden durch Vergleich mit Peroxidase Standardlösungen. Dabei wird häufig Meerrettichperoxidase verwendet. Die Vergleichbarkeit mit Lactoperoxidase ist jedoch nicht gewährleistet.

In EP 0 832 985 wird ein auf einem festen Träger durchgeführtes Verfahren zum Nachweis von Lactoperoxidase bzw. alkalischer Phosphatase beschrieben. Durch die trockenchemische Durchführung werden einige der oben angeführten Nachteile vermieden. Die Versuchsdurchführung ist jedoch mit einer Dauer von 20 bis 60 Minuten sehr zeitaufwendig. Zudem ist die Nachweisempfindlichkeit des Verfahrens, wie auch bei den meisten naßchemischen Methoden, zu gering. Als eine für den Anwender ausreichende Empfindlichkeit ist der Nachweis von mindestens 1 % Rohmilch in peroxidasefreier Milch (z.B. UHT-Milch) anzustreben.

Der vorliegenden Erfindung liegt daher die Aufgabe zu Grunde, ein Verfahren zur Bestimmung von Lactoperoxidase in Milch bzw. Milchprodukten zur Verfügung zu stellen, das einfach und schnell in der Ausführung, spezifisch und kostengünstig ist, und gleichzeitig die geforderte Empfindlichkeit gewährleistet. Zudem sollte sich das erfindungsgemäße Verfahren zur Auswertung mit einem Reflektometer eignen.

Überraschenderweise wurde gefunden, daß man ein spezifisches und hochsensitives Nachweissystem für Lactoperoxidase in Gegenwart von Wasserstoffperoxid erhält, wenn man die üblichen Substrate und Puffer für den Peroxidase-Nachweis einsetzt und zusätzlich eine bestimmte Menge an lodidionen zusetzt.

Während der Zusatz von lodidionen eine deutliche Verringerung der Nachweissensitivität für Meerrettichperoxidase zur Folge hat, bewirkt der Zusatz von lodidionen in Bezug auf Lactoperoxidase eine deutliche Steigerung der Nachweisempfindlichkeit.

Der Zusatz von lodidionen muß dabei genau abgestimmt sein, da überhöhte Mengen zu einer unerwünschten Nebenreaktion führen. Ohne Substrat wird auch in Gegenwart sehr hoher lodidkonzentrationen kein Farbumschlag beobachtet. Der gezielte Zusatz einer bestimmten Menge an lodidionen ermöglicht somit die Bereitstellung eines ausreichend sensitiven Meßsystems für Lactoperoxidase in Milch bzw. Milchprodukten. Um eine schnelle Versuchsdurchführung und einen geringen Reagenzienverbrauch sicherzustellen, wird das erfindungsgemäße Verfahren bevorzugt trockenchemisch durchgeführt.

Gegenstand der Erfindung ist ein Mittel zum Nachweis von Lactoperoxidase, bestehend aus einem Träger, auf den zumindest ein Peroxidasesubstrat und ein lodid aufgebracht sind, wobei die Iodid-Konzentration in der Lösung, mit der der Träger imprägniert wurde, zwischen 1,5 und 8 mmol/l liegt..

In einer bevorzugten Ausführungsform des erfindungsgemäßen Mittels befindet sich als Substrat 1,4-Phenylendiamin, 2,2'-azino-bis-(3-ethylbenzthiazol-6-sutfonsäure) oder 3,3',5,5'-Tetramethylbenzidin auf dem Träger.

Gegenstand der Erfindung ist ein Verfahren zur Bestimmung von Lactoperoxidase in Milch bzw. Milchprodukten, das durch folgende Reaktionsschritte gekennzeichnet ist:
a) Bereitstellen einer Probe enthaltend Milch oder Milchprodukte
b) Bereitstellen eines Trägers, auf den zumindest ein PeroxidaseSubstrat und ein lodid aufgebracht sind, wobei die Iodid-Konzentration in der Lösung, mit der der Träger imprägniert wurde, zwischen 1,5 und 8 mmol/l liegt;
c) optional, falls notwendig, Zugabe von auf dem Träger instabilen Reagenzien, wie beispielsweise eines Peroxidbildners, zu der Probe;
d) kurzes Eintauchen des Trägers in die Probe;
e) Abwarten der Farbreaktion;
f) Nachweis der Farbreaktion.

In einer bevorzugten Ausführungsform des erfindungsgemäßen Verfahrens wird in Schritt b) der Peroxidbildner Wasserstoffperoxid zugegeben.

In einer bevorzugten Ausführungsform des erfindungsgemäßen Verfahrens erfolgt der Nachweis der Farbreaktion in Schritt e) reflektometrisch.
Gegenstand der vorliegenden Erfindung ist weiterhin die Verwendung des erfindungsgemäßen Mittels und Verfahrens zur Bestimmung der Aktivität von Lactoperoxidase in hocherhitzter Milch.

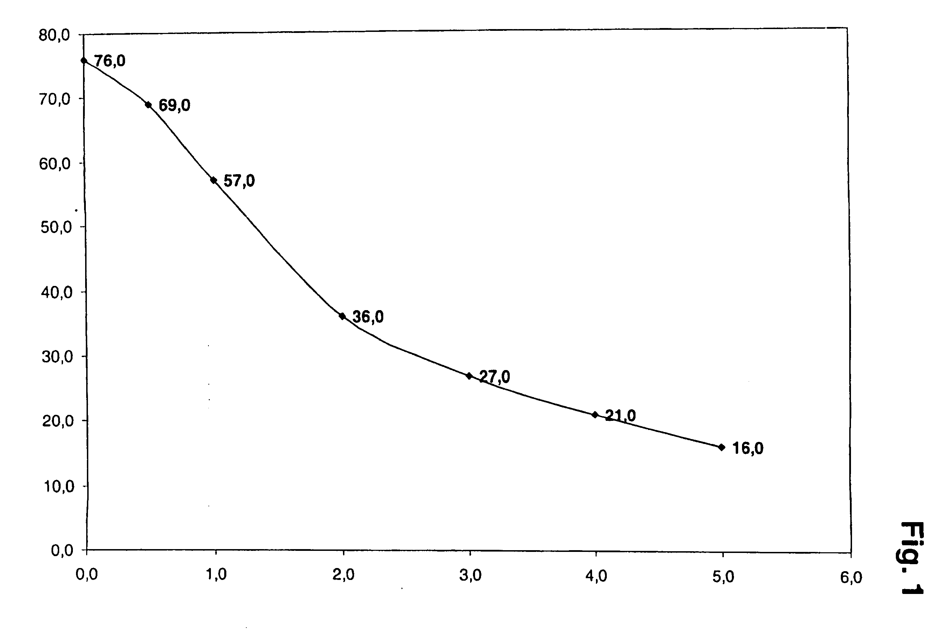

Die Abbildungen 1 bis 4 sind in den Beispielen 1 bis 4 näher erläutert.

Wesentliche Voraussetzung für das Erzielen der gewünschten Nachweisempfindlichkeit ist der Zusatz von lodidionen. Geeignete lodide sind beispielsweise die Alkaliiodide und Ammoniumiodid, vorzugsweise Lithiumiodid. Die optimale lodidkonzentration muß auf die Bedingungen des Nachweissystems abgestimmt sein. Der Fachmann ist in der Lage, mit wenigen Versuchen diese Konzentration zu ermitteln (siehe dazu Beispiel 2). Typischerweise liegt die lodidkonzentration in der Lösung, mit der der Träger imprägniert wird, zwischen 1,5 und 8 mmol/l, besonders bevorzugt zwischen 3 und 4 mmol/l.

Als Substrate können alle in der Literatur zum Nachweis von Peroxidasen bekannten Reagenzien bzw. Reagenzkombinationen eingesetzt werden, die zu geeigneten Farbumschlägen führen und einen sensitiven Nachweis ermöglichen. Bevorzugt sind dies 1,4-Phenylendiamin, 2,2'-azino-bis-(3-ethylbenzthiazol-6-sulfonsäure) und besonders bevorzugt 3,3',5,5'-Tetramethylbenzidin.

Als Peroxidbildner werden erfindungsgemäß für derartige Anwendungen bekannte Substanzen wie z.B. Wasserstoffperoxid-Harnstoff und besonders bevorzugt Wasserstoffperoxid eingesetzt. Ebenso kann Wasserstoffperoxid in situ, beispielsweise durch die bekannte Reaktion von Glucose mit Glucose-Oxidase, erzeugt werden.

Der pH-Wert des Nachweissystems muß so aufeinander abgestimmt sein, daß einerseits das Enzym seine höchste Aktivität entfalten kann, andererseits der für die Farbreaktion (abhängig vom Substrat) günstigste pH-Wert vorliegt. Für das erfindungsgemäße Verfahren werden daher, besonders bei Milchprodukten, dem Reagenzsystem gegebenenfalls auch Puffersubstanzen zugesetzt, um die Reaktion in einem optimalen pH-Bereich ablaufen zu lassen. Als Probenpuffer kommen solche in Frage, die den pH-Wert auf ca. 5,0 - 7,0 einstellen. Bevorzugt ist der pH-Bereich 6,0 - 7,0. Mögliche Puffer sind z.B. Phosphatpuffer.

Das Testsystem liegt vorzugsweise in Form einer imprägnierten Matrix vor, d.h. weitgehend alle für den Nachweis notwendigen Reagenzien (Substrat, lodid, Peroxidbildner und optional Puffer), gegebenenfalls auch Stabilisatoren und Lösungsvermittler, sind in einen saugfähigen Träger eingebettet. Der Träger wird direkt in die zu untersuchende Probe eingetaucht und die Farbreaktion geeignet verfolgt. In Ausnahmefällen müssen Substanzen, die z.B. auf dem Träger nicht ausreichend stabil sind, als Lösung zugesetzt werden. Der Fachmann ist in der Lage, derartige Reagenzien in geeigneten Konzentrationen zuzusetzen. Beispielsweise wird bei der Verwendung von Wasserstoffperoxid eine entsprechende Lösung kurz vor Eintauchen des Teststäbchens zu der Probe gegeben (Schritt b) des erfindungsgemäßen Verfahrens). Bevorzugt werden jedoch alle Reagenzien auf dem Träger eingebettet. In diesem Fall entfällt Schritt b) des erfindungsgemäßen Verfahrens.

Als saugfähige Träger können alle Materialien verwendet werden, die üblicherweise für solche Tests in Gebrauch sind. Am weitesten verbreitet ist die Verwendung von Filterpapier, jedoch können auch andere saugfähige Cellulose- oder Kunststoffprodukte eingesetzt werden. Die saugfähigen Träger werden in bekannter Weise mit Tränklösungen imprägniert, die die zur Bestimmung von Peroxidase notwendigen Reagenzien enthalten. Die getränkten und getrockneten Papiere können geeignet zugeschnitten und in bekannter Weise auf Trägerfolien aufgeklebt bzw. aufgesiegelt werden.

Der Nachweis der Farbreaktion erfolgt spektralphotometrisch, visuell oder bevorzugt reflektometrisch.

Falls nicht alle zum Peroxidasenachweis notwendigen Substanzen auf dem erfindungsgemäßen Teststreifen vorliegen, kann ein Testkit zusammengestellt werden, der neben dem Teststreifen die Substanzen oder Lösungen der Substanzen enthält, die der Probe zusätzlich zugesetzt werden müssen.

Zur Durchführung des erfindungsgemäßen Verfahrens wird die zu untersuchende Milchprobe entsprechend der zu erwartenden Aktivität der Peroxidase mit Pufferlösung oder bevorzugt mit deionisiertem Wasser (VE-Wasser) verdünnt. Typischerweise ist eine 1:5 Verdünnung ausreichend. Anschließend wird die Probe bevorzugt mit einer Lösung versetzt, die Wasserstoffperoxid enthält. Typischerweise beträgt der Gehalt an Wasserstoffperoxid in der Probenlösung zwischen 0,005 und 1%, bevorzugt zwischen 0,01 und 0,1%.

Nach kurzem Durchmischen wird sofort ein geeignet präpariertes Teststäbchen kurz in die Lösung getaucht. Anschließend wird in der Regel überschüssige Lösung leicht von dem Träger abgestreift und die Farbreaktion abgewartet. Nach Ablauf einer bestimmten Reaktionszeit, typischerweise zwischen 1 und 5 Minuten, wird das Teststäbchen bevorzugt reflektometrisch ausgewertet. Bei der Verwendung eines Peroxidbildners, der auf das Teststäbchen aufgebracht werden kann, entfällt die vorherige Zugabe von Wasserstoffperoxid.

Zur Durchführung des erfindungsgemäßen Verfahrens werden typischerweise ca. 3-5 Minuten benötigt.

Bei der Verwendung des erfindungsgemäßen Verfahrens wurde festgestellt, daß übliche Methoden zur Kalibration nicht ausreichend genau sind. Beispielsweise hat sich eine Kalibration mit Lactoperoxidase-Standardlösungen (hergestellt durch Auflösen von käuflicher, gefriergetrockneter Lactoperoxidase) wie häufig in der Literatur beschrieben (z.B. EP 0 832 985), als nicht mit der Praxis vergleichbar und damit als ungeeignet erwiesen, da bei dieser Vorgehensweise deutlich andere Lactoperoxidaseaktivitäten resultieren. Zur Auswertung der Farbreaktionen des erfindungsgemäßen Verfahrens werden deshalb Standardlösungen aus peroxidasefreier Milch hergestellt, die mit unterschiedlichen Mengen einer frischen Rohmilch versetzt wird.

Weiterhin wurde festgestellt, daß für das erfindungsgemäße Verfahren eine Kalibration mit Merettichperoxidase ungeeignet ist. Während durch das erfindungsgemäße Verfahren die Nachweisempfindlichkeit für Lactoperoxidase stark zunimmt, tritt für Merettichperoxidase ein gegenteiliger Effekt auf.

Es wurde jedoch gefunden, daß das erfindungsgemäße Verfahren ebenfalls geeignet ist, den Peroxidase-Gehalt von bestimmten Gemüsen bzw. von Getreide zu untersuchen. Vor dem Einfrieren muß Gemüse einer Hitzebehandlung (Blanchieren) unterzogen werden, insbesondere mit dem Ziel, unerwünschte Enzymaktivitäten zu beseitigen und die Lagerstabilität zu erhöhen. Die Wirksamkeit dieser Behandlung kann mittels eines Peroxidase-Tests in geeigneter Weise verfolgt werden (B. P. Wassermann and J.D. Wagner, 13 (2), 1985, S. 84-85). Ebenso geeignet ist der Nachweis mittels des erfindungsgemäßen Verfahrens. Genauere Angaben finden sich in Beispiel 5.

Somit bietet das erfindungsgemäße Verfahren die Möglichkeit zum schnellen und empfindlichen Nachweis bestimmter Peroxidasen, besonders Lactoperoxidase. Mit dem amtlich zugelassenen Traventol^{®}-Reagenz können, ein geschultes Auge vorausgesetzt, z.B. nur ca. 3% Rohmilch in peroxidasefreier Milch nachgewiesen werden (E. Olszewski u. H. Reuter, Z. Lebensm. Unters. Forsch. 194 (1992), S. 235 - 2390). Mit dem erfindungsgemäßen Verfahren können typischerweise Gehalte von Rohmilch in peroxidasefreier Milch nachgewiesen werden, die deutlich unter 1 %, oft sogar unter 0,5%, liegen.

Durch die Einbettung der Reagenzien auf einem Träger wird die Durchführung des erfindungsgemäßen Peroxidase-Nachweises stark vereinfacht und die Menge an Reagenzien reduziert.

Der vorzugsweise reflektometrische Nachweis erfordert keinen großen apparativen Aufwand und liefert zugleich genauere Ergebnisse als eine visuelle Auswertung.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen, insbesondere der korrespondierenden Anmeldung DE 199 57 367, eingereicht am 29.11.1999, ist durch Bezugnahme in diese Anmeldung eingeführt.

### Beispiele

Sofern nicht anders ausgewiesen wurden alle Reagenzien von Merck KGaA bezogen.

### 1. Bestimmung von Peroxidase mit Testsstreifen - reflektometrische Auswertung der Reaktionsfarbe:

### Herstellung der Teststreifen:

Ein Filterpapier (Fa. Binzer, 1450 CV säuregewaschen) wird mit nachfolgender Tränklösung getränkt und nach der Tränkung mit warmer Luft getrocknet. Das Papier wird mit Schmelzkleber (z.B. Kleber Technomelt Q ^{®} 3273 der Firma Henkel, Düsseldorf) auf eine weiße Trägerfolie aufgesiegelt und geeignet in Streifen geschnitten, so daß eine Reaktionszone von ca. 6 mm x 8 mm resultiert.

### Zusammensetzung der Tränklösung:

In 1 I Ethanol (96%) werden 5,0 g Malonsäure, 2,0 g 3,3',5,5'-Tetramethylbenzidin und 0,45 g Lithiumiodid gelöst.

### Herstellung der Standardlösungen:

Peroxidasefreie Milch, hergestellt durch Erhitzen frischer Rohmilch, wird mit unterschiedlichen Mengen einer frischen Rohmilch versetzt, so daß Standardlösungen im Bereich 0-5% Rohmilch bezogen auf peroxidasefreie Milch resultieren.

Eine Kalibration mit Lactoperoxidase-Standardlösungen (hergestellt durch Auflösen von käuflicher, gefriergetrockneter Lactoperoxidase in peroxidasefreier Milch) wie häufig in der Literatur beschrieben (z.B. EP 0 832 985 A2), hat sich als nicht mit der Praxis vergleichbar und damit als ungeeignet erwiesen, da bei dieser Vorgehensweise deutlich andere Lactoperoxidaseaktivitäten resultieren.

### Analyse:

Die erhaltenen Milchproben werden zur Analyse 1:5 mit VE-Wasser verdünnt. 5 ml der verdünnten Probe werden mit 5 Tropfen (ca. 0,25 ml) 0,3%iger Wasserstoffperoxidlösung versetzt. Die Teststäbchen werden umgehend in die Proben eingetaucht.

In Abhängigkeit von der Konzentration an Peroxidase entstehen unterschiedlich intensive Blaufärbungen, die visuell durch Vergleich mit einer Farbkarte ausgewertet werden können. Zur quantitativen Auswertung werden die Teststreifen nach exakt 3 Minuten in einem kleinen Handreflektometer auf Diodenbasis (Reflektometer RQflex^{®} der Firma Merck KGaA) ausgewertet.

Den Zusammenhang zwischen der gemessenen relativen Remission (%) und den Anteilen an Rohmilch zeigt Abbildung 1. Auf der Abszisse ist der Anteil an Rohmilch in % aufgetragen, auf der Ordinate die relative Remission in %. Rohmilchanteile von 0,5% führen zu einer deutlichen Blaufärbung und können mit dem Verfahren sicher nachgewiesen werden. Das Verfahren ist damit deutlich empfindlicher als bestehende Verfahren.

### 2. Einfluß des lodidgehalts auf die Sensitivität des Nachweissystems für Lactoperoxidase:

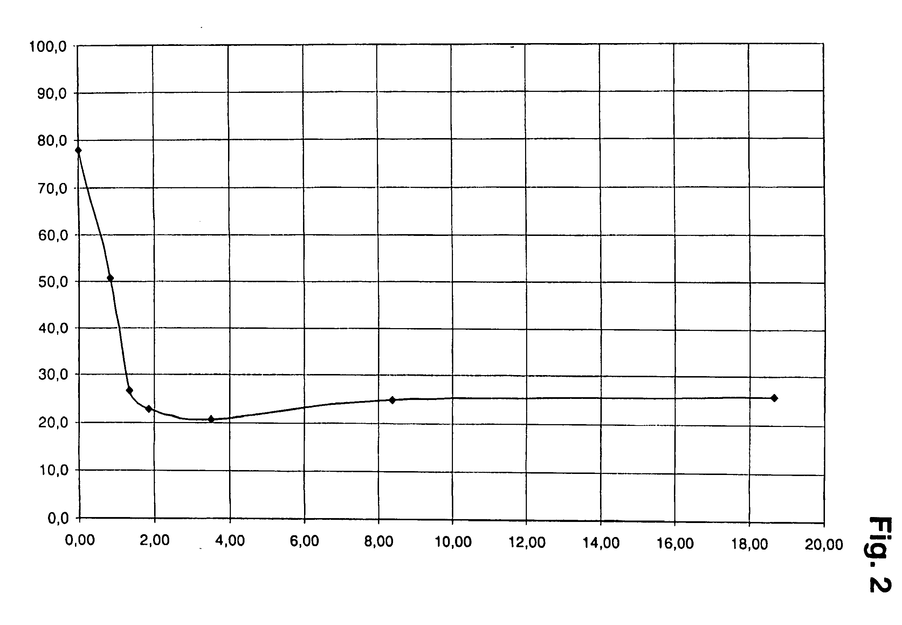

Die Teststäbchen wurden gemäß Beispiel 1 angefertigt und ausgewertet. Dabei wurde die lodidkonzentration variiert. Der Anteil an Rohmilch und damit der Peroxidasegehalt wurde konstant bei 5% gehalten. Ein Zusatz an lodid führt, wie aus Abbildung 2 ersichtlich, zu einer deutlichen Steigerung der Nachweisempfindlichkeit. Bei lodidkonzentrationen über ca. 8 mmol/l wird die Reaktionsfarbe infolge einer unerwünschten Nebenreaktion von einer unspezifischen Blaufärbung überlagert. In Abbildung 2 ist auf der Abszisse die lodidkonzentration in der Tränklösung, auf der Ordinate die Remission in % aufgetragen.

### 3. Einfluß des lodidgehalts auf die Sensitivität des Nachweissystems für Meerrettichperoxidase:

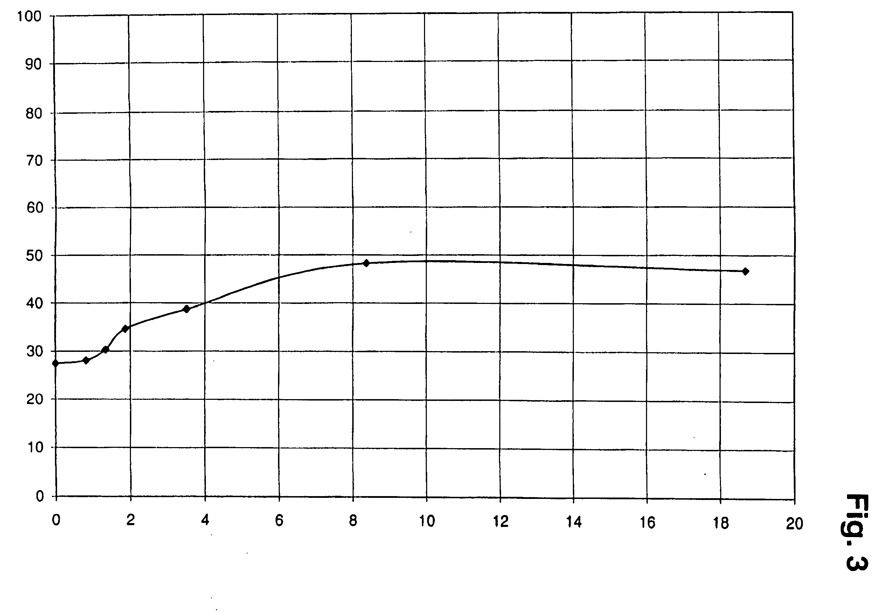

Teststäbchen aus Beispiel 2 mit unterschiedlichen lodidkonzentrationen wurden in eine wäßrige, mit Meerrettichperoxidase dotierte Lösung getaucht und ausgewertet. Wie der Abbildung 3 zu entnehmen ist, führt ein lodidzusatz zu einer Verringerung der Nachweisempfindlichkeit des Testsystems in Bezug auf Meerrettichperoxidase. In Abbildung 3 ist auf der Abszisse die Iodidkonzentration in der Tränklösung, auf der Ordinate die Remission in % aufgetragen.

### 4. Praxistest an verschiedenen Proben hitzebehandelter Milch:

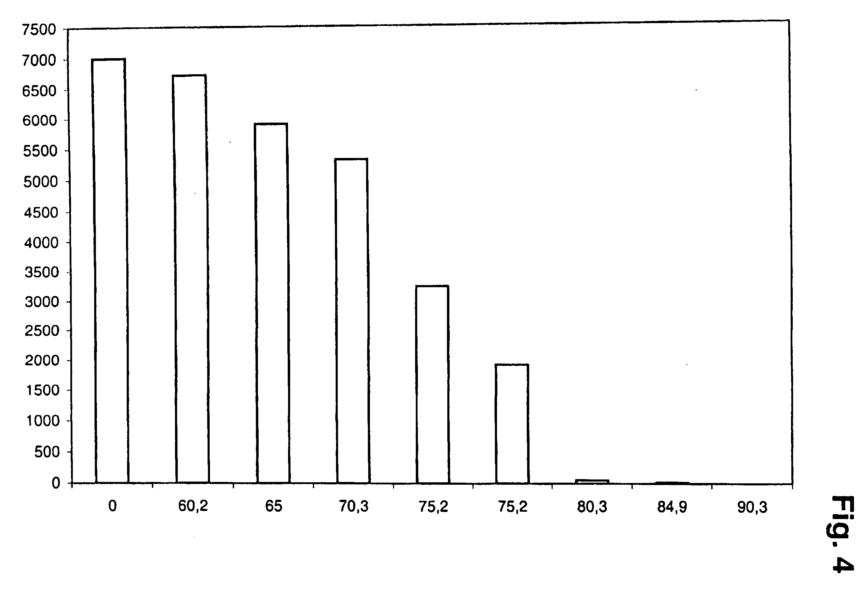

Tesstäbchen aus Beispiel 1 wurden in unterschiedlich hitzebehandelte Milch eingetaucht und gemäß Beispiel 1 analysiert. Proben mit höherer Aktivität als im Meßbereich wurden zusätzlich mit peroxidasefreier Milch verdünnt. Die Ergebnisse sind in Abbildung 4 dargestellt. Entlang der Abszisse ist die Erhitzungstemperatur in °C aufgetragen. Die Heißhaltezeit betrug jeweils 20,2 Sekunden. Um den zweiten Meßpunkt bei 75,2°C mit niedrigerer Aktivität zu erhalten, wurde die Heißhaltezeit auf 76,5 Sekunden verlängert. Die Ordinate gibt die verbleibende Aktivität der Lactoperoxidase in U/l an.

### 5. Praxistest an Gemüse und Getreide:

### Analyse von Kartoffeln und Karotten

10 g des Untersuchungsmaterials werden mit 40 ml VE-Wasser versetzt, in einer Moulinette zerkleinert und das Filtrat je nach Bedarf (abhängig von Peroxidase-Aktivität) weiterverdünnt. Die Probelösung wird mit 5 Tropfen (ca. 0,25 ml) 0,3%iger Wasserstoffperoxidlösung versetzt und die Teststäbchen werden umgehend in die Probe eingetaucht.

### Ergebnis:

Am Beispiel einer Kartoffel wurde nach Gesamtverdünnung um Faktor 1:25 eine Färbung erhalten, die ca. einem Wert für Milch von 5% Rohmilch in peroxidasefreier Milch entspricht (siehe Beispiel 1). Am Beispiel einer Möhre wurde nach Gesamtverdünnung um Faktor 1:25 eine Färbung erhalten, die ca. einem Wert für Milch von 2,5% Rohmilch in peroxidasefreier Milch entspricht (siehe Beispiel 1).

## Patentansprüche

1. Mittel zum Nachweis von Lactoperoxidase in Milch bzw. Milchprodukten, bestehend aus einem Träger, auf den zumindest ein Peroxidasesubstrat und ein lodid aufgebracht sind, wobei die lodid-Konzentration in der Lösung, mit der der Träger imprägniert wurde, zwischen 1,5 und 8 mmol/l liegt.

2. Mittel zum Nachweis von Lactoperoxidase nach Anspruch 1, **dadurch gekennzeichnet, daß** sich als Substrat 1,4-Phenylendiamin, 2,2'-azino-bis(3-ethylbenzthiazol-6-sulfonsäure) oder 3,3',5,5'-Tetramethylbenzidin auf dem Träger befindet.

3. Verfahren zur Bestimmung von Lactoperoxidase in Milch bzw. Milchprodukten, **gekennzeichnet durch** folgende Reaktionsschritte:
a) Bereitstellen einer Probe enthaltend Milch oder Milchprodukte
b) Bereitstellen eines Trägers, auf den zumindest ein PeroxidaseSubstrat und ein lodid aufgebracht sind, wobei die lodid-Konzentration in der Lösung, mit der der Träger imprägniert wurde, zwischen 1,5 und 8 mmol/l liegt;
c) optionale Zugabe von nicht trägerstabilen Reagenzien zu der Probe;
d) kurzes Eintauchen des Trägers in die Probe;
e) Abwarten der Farbreaktion;
f) Nachweis der Farbreaktion.

4. Verfahren nach Anspruch 3, **dadurch gekennzeichnet, daß** in Schritt c) der Peroxidbildner Wasserstoffperoxid zugegeben wird.

5. Verfahren nach einem der Ansprüche 3 oder 4, **dadurch gekennzeichnet, daß** der Nachweis der Farbreaktion in Schritt f) reflektometrisch erfolgt.

6. Verwendung des Mittels nach einem der Ansprüche 1 oder 2 zur Bestimmung der Aktivität von Lactoperoxidase in hocherhitzter Milch.

7. Verwendung des Verfahrens nach einem der Ansprüche 3 bis 5 zur Bestimmung der Aktivität von Lactoperoxidase in hocherhitzter Milch.

## Claims

1. Composition for the detection of lactoperoxidase in milk or milk products, consisting of a support to which at least one peroxidase substrate and an iodide have been applied, where the iodide concentration in the solution with which the support has been impregnated is between 1.5 and 8 mmol/l.

2. Composition for the detection of lactoperoxidase according to Claim 1, **characterised in that** the substrate located on the support is 1,4-phenylenediamine, 2,2'-azinobis(3-ethylbenzothiazole-6-sulfonic acid) or 3,3',5,5'-tetramethylbenzidine.

3. Method for the determination of lactoperoxidase in milk or milk products, **characterised by** the following reaction steps:
a) provision of a sample comprising milk or milk products
b) provision of a support to which at least one peroxidase substrate and an iodide have been applied, where the iodide concentration in the solution with which the support has been impregnated is between 1.5 and 8 mmol/l;
c) optional addition of non-support-stable reagents to the sample;
d) brief immersion of the support into the sample;
e) waiting for the colour reaction;
f) detection of the colour reaction.

4. Method according to Claim 3, **characterised in that** the peroxide former added in step c) is hydrogen peroxide.

5. Method according to one of Claims 3 and 4, **characterised in that** the detection of the colour reaction in step f) is carried out by reflectometry.

6. Use of the composition according to one of Claims 1 and 2 for the determination of the activity of lactoperoxidase in ultra-heat-treated milk.

7. Use of the method according to one of Claims 3 to 5 for the determination of the activity of lactoperoxidase in ultra-heat-treated milk.

## Revendications

1. Composition pour la détection de lactoperoxydase dans le lait ou les produits laitiers, constituée par un support sur lequel au moins un substrat de peroxydase et un iodure ont été appliqués, où la concentration en iodure dans la solution dont le support a été imprégné est entre 1,5 et 8 mmol/l.

2. Composition pour la détection de lactoperoxydase selon la revendication 1, **caractérisée en ce que** le substrat localisé sur le support est 1,4-phénylènediamine, 2,2'-azinobis(acide 3-éthylbenzothiazole-6-sulfonique) ou 3,3',5,5'-tétraméthylbenzidine.

3. Procédé pour la détermination de lactoperoxydase dans le lait ou les produits laitiers, **caractérisé par** les étapes de réaction qui suivent:
a) fourniture d'un échantillon comprenant du lait ou des produits laitiers
b) fourniture d'un support sur lequel au moins un substrat de peroxydase et un iodure ont été appliqués, où la concentration en iodure dans la solution dont le support a été imprégné est entre 1,5 et 8 mmol/l;
c) optionnel ajout de réactifs non stables pour le support à l'échantillon;
d) brève immersion du support dans l'échantillon;
e) attente de la réaction colorée;
f) détection de la réaction colorée.

4. Procédé selon la revendication 3, **caractérisé en ce que** le peroxyde ajouté précédemment au niveau de l'étape c) est du peroxyde d'hydrogène.

5. Procédé selon l'une des revendications 3 et 4, **caractérisé en ce que** la détection de la réaction coloré au niveau de l'étape f) est mise en oeuvre par réflectométrie.

6. Utilisation de la composition selon l'une des revendications 1 et 2 pour la détermination de l'activité de lactoperoxydase dans du lait ultra-haute température.

7. Utilisation du procédé selon l'une des revendications 3 à 5 pour la détermination de l'activité de lactoperoxydase dans du lait ultra-haute température.
